# EUROPÄISCHE PATENTSCHRIFT

(11) **EP 0 510 154 B1**
(45) Veröffentlichungstag und Bekanntmachung des Hinweises auf die Patenterteilung: **10.05.1995**
(21) Anmeldenummer: 91919671.7
(22) Anmeldetag: 08.11.1991
(51) Int. Cl.: C07C 405/00, A61K 31/557

(54) **9-HALOGEN-11(Beta)-HYDROXY-PROSTAGLANDINDERIVATE, VERFAHREN ZU IHRER HERSTELLUNG UND IHRE VERWENDUNG ALS ARZNEIMITTEL**
9-HALOGEN-11(Beta)-HYDROXY PROSTAGLANDIN DERIVATIVE, PROCESS FOR PRODUCING IT AND ITS USE AS A MEDICAMENT
DERIVES DE LA 9-HALOGENE-11(Beta)-HYDROXYPROSTAGLANDINE, PROCEDES DE FABRICATION ET APPLICATION COMME MEDICAMENT

(30) Priorität: 09.11.1990 DE 4036140
(43) Veröffentlichungstag der Anmeldung: 28.10.1992
(73) Patentinhaber: SCHERING AKTIENGESELLSCHAFT, 13342 Berlin (DE)
(72) Erfinder: BUCHMANN, Bernd, D-1000 Berlin 21 (DE); SKUBALLA, Werner, D-1000 Berlin 37 (DE); THIERAUCH, Karl-Heinz, D-1000 Berlin 37 (DE); VERHALLEN, Peter, D-1000 Berlin 28 (DE)
(86) Internationale Anmeldenummer: DE9100881
(87) Internationale Veröffentlichungsnummer: WO9208697

(56) Entgegenhaltungen:
- EP-A- 0 030 377
- EP-A-02 999 914
- WO-A-89/03384
- Prostaglandins, vol. 11, No. 1 January 1976, W.L. MILLER et al.: "Relative biological activity of certain prostaglandins and their enantiomers", pages 77-84, see page 80, table 1

## Beschreibung

Gegenstand der vorliegenden Erfindung sind neue 9-Halogen-11β-hydroxyprostaglandinderivate, Verfahren zu ihrer Herstellung sowie ihre Verwendung als Arzneimittel.

Aus dem sehr umfangreichen Stand der Technik der Prostaglandine und ihrer Analoga weiß man, daß diese Stoffklasse aufgrund ihrer biologischen und pharmakologischen Eigenschaften zur Behandlung von Säugetieren, einschließlich des Menschen, geeignet ist, Ihre Verwendung als Arzneimittel stößt jedoch häufig auf Schwierigkeiten. Die meisten natürlichen Prostaglandine besitzen eine für therapeutische Zwecke zu kurze Wirkungsdauer, da sie zu rasch durch verschiedene enzymatische Prozesse metabolisch abgebaut werden. Alle Strukturveränderungen haben das Ziel, die Wirkungsdauer sowie die Selektivität der Wirksamkeit zu steigern.

Es wurde nun gefunden, daß die neuen 9-Halogen-11β-hydroxy-prostaglandinderivate eine hervorragende Wirkungsspezifität, bessere Wirksamkeit, längere Wirkungsdauer als natürliche Prostaglandine und deren Derivate besitzen und besonders für die orale Applikation geeignet sind.

Die Erfindung betrifft 9-Halogen-11β-hydroxy-prostanderivate der Formel I 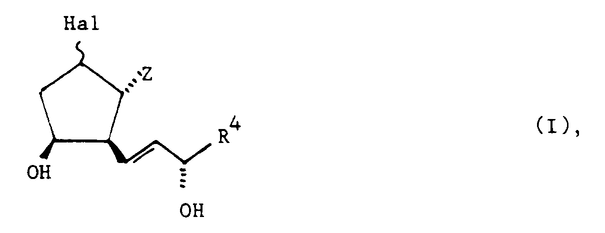
worin Z die Reste 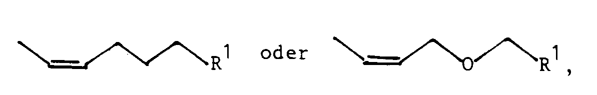
Hal ein α- oder β-ständiges Chlor- oder Fluoratom,
R¹ den Rest CH₂OH oder 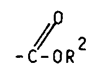
mit R² in der Bedeutung eines Wasserstoffatoms, eines Alkyl-, Cycloalkyl-, Aryl- oder heterocyclischen Restes oder R¹ den Rest 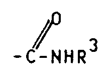
mit R³ in der Bedeutung eines Säurerestes oder des Restes R² und R⁴ eine Cycloalkylgruppe, und falls R² die Bedeutung eines Wasserstoffatoms hat, deren Salze mit physiologisch verträglichen Basen bedeuten und deren Cyclodextrinclathrate.

Als Alkylgruppen R² sind gerade oder verzweigte Alkylgruppen mit 1-10 C-Atomen zu betrachten, wie beispielsweise Methyl, Ethyl, Propyl, Butyl, Isobutyl, tert.-Butyl, Pentyl, Neopentyl, Hexyl, Heptyl, Decyl. Die Alkylgruppen R² können gegebenenfalls ein- bis mehrfach substituiert sein durch Halogenatome, Alkoxygruppen, gegebenenfalls substituierte Aryl- bzw. Aroylgruppen, Dialkylamino und Trialkylammonium, wobei die einfache Substitution bevorzugt sein soll. Als Substituenten seien beispielsweise genannt Fluor, Chlor oder Brom, Phenyl, Dimethylamino, Diethylamino, Methoxy, Ethoxy. Als bevorzugte Alkylgruppen R² sind solche mit 1-4 C-Atomen, wie z.B. Methyl, Ethyl, Propyl, Dimethylaminopropyl, Isobutyl, Butyl zu nennen.

Als Arylgruppen R² kommen sowohl substituierte wie auch unsubstituierte Arylgruppen in Betracht, wie beispielsweise Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen, eine Chlormethyl-, Fluormethyl-, Trifluormethyl-, Carboxyl-, Hydroxy- oder Alkoxygruppe mit 1-4 C-Atomen. Bevorzugt sind die Substituenten in 3- und 4-Stellung am Phenylring, zum Beispiel durch Fluor, Chlor, Alkoxy oder Trifluormethyl oder in 4-Stellung durch Hydroxy.

Die Cycloalkylgruppe R² kann im Ring 3-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl-, Cyclohexyl, Methylcyclohexyl und Adamantyl.

Als heterocyclische Gruppen R² kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pridyl, 4-Pyridyl, Oxazolyl, Thiazolyl, Pyrimidinyl, Pyridazinyl, Pyrazinyl, 3-Furyl, 3-Thienyl, 2-Tetrazolyl u.a.

Als Säurerest R³ kommen physiologisch verträgliche Säurereste in Frage. Als Säuren sind organische Carbonsäuren und Sulfonsäuren mit 1-15 Kohlenstoffatomen geeignet, die der aliphatischen, cycloaliphatischen, aromatischen, aromatisch-aliphatischen und heterocyclischen Reihe angehören. Diese Säuren können gesättigte, ungesättigte und/oder mehrbasisch und/oder in üblicher Weise substituiert sein. Als Beispiele für Substituenten seien Alkyl-, Hydroxy-, Alkoxy-, Oxo- oder Aminogruppen oder Halogenatome erwähnt. Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyessigsäure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy-, Alkoxy- oder Carboxy-Gruppen substutierte Benzoesäuren, Nikotinsäure, Isonikotinsäure, Furan-2-carbonsäure, Cyclopentylpropionsäure. Als bevorzugte Acylreste werden solche mit bis zu 10 Kohlenstoffatomen betrachtet. Als Sulfonsäuren kommen beispielsweise Alkansulfonsäuren mit 1-10 C-Atomen wie z.B. Methansulfonsäure, Ethansulfonsäure, Isopropansulfonsäure und Butansulfonsäure sowie β-Chlorethansulfonsäure, Cyclopentansulfonsäure, Cyclohexansulfonsäure, Benzolsulfonsäure, p-Toluolsulfonsäure, p-Chlorbenzolsulfonsäure, N.N-Dimethylaminosulfonsäure, N.N-Diethylaminosulfonsäure, N.N-Bis- (β-chlorethyl)-aminosulfonsäure, N.N-Diisobutylaminosulfonsäure, N.N-Dibutylaminosulfonsäure, Pyrrolidino-, Piperidino-, Pipepazino-, N-Methylpiperazino- und Morpholinosulfonsäure in Frage. Besonders bevorzugt sind Acylreste bzw. Alkansulfonsäurereste mit 1-4 C-Atomen.

Die Cycloalkylgruppe R⁴ kann im Ring 3-10, vorzugsweise 3-6 Kohlenstoffatome enthalten. Die Ringe können durch Alkylgruppen mit 1-4 Kohlenstoffatomen substituiert sein. Beispielsweise genannt seien Cyclopropyl, Cyclobutyl, Cyclopentyl, Cyclohexyl, Methyl-cyclohexyl insbesondere 4-Methylcyclohexyl, 4-Ethylcyclohexyl, 4-Propylcyclohexyl und Adamantyl.

Zur Salzbildung sind anorganische und organische Basen geeignet, wie sie dem Fachmann zur Bildung physiologisch vertraglicher Salze bekannt sind. Beispielsweise seien genannt Alkalihydroxide, wie Natrium- und Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin usw.

Die Erfindung betrifft außerdem ein Verfahren zur Herstellung der erfindungsgemäßen 9-Halogen-11β-hydroxy-prostanderivate der Formel I, dadurch gekennzeichnet, daß man in an sich bekannter Weise eine Verbindung der Formel II 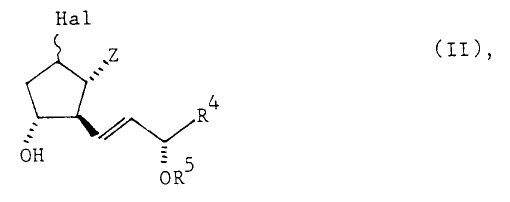
worin Z, Hal und R⁴ die bereits oben ausgegebenen Bedeutungen haben und R⁵ ein Silylreste sein kann, beispielsweise seien genannt Trimethylsilyl, Dimethyltert.-butylsilyl, Dimethyl-hexylsilyl, Diphenyl-tert.-butylsilyl und Tribenzylsilyl, durch eine Oxidation mit Chromsaure oder einem Chromsäurederivat intermediar in ein Keton überführt, dieses anschließend durch eine Reduktion in ein Gemisch der epimeren Alkohole umgesetzt und nach Reinigung gegebenenfalls in beliebiger Reihenfolge vorhandene Schutzgruppen abspaltet und/oder eine veresterte Carboxylgruppe 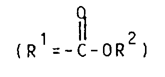
verseift und/oder reduziert wird.

Die Umsetzung der Verbindungen der Formel II zu den Verbindungen der Formel I erfolgt zunächst durch die Oxidation der freien Hydroxygruppe nach den dem Fachmann bekannten Methoden. Als Oxidationsmittel können beispielsweise dienen: Jones-Reagenz (J.Chem.Soc. 1953, 2555), Pyridiniumdichromat (Tetrahedron Letters 1979, 399), Collins-Reagenz (Tetrahedron Letters 1968, 3363), Pyridiniumchlorochromat (Tetrahedron Letters 1975, 2647), bevorzugt wird die Methode mit Pyridiniumchlorochromat.

Die Oxidation mikt Jones-Reagenz wird bei Temperaturen von -40°C bis- +40°C, vorzugsweise -20°C bis 10°C ins Aceton als Lösungsmittel durchgeführt. Die Oxidation mit Pyridiniumdichromat wird bei Temperaturen von 0°C bis 100°C, vorzugsweise 20°C bis 40°C in einem gegen das Oxidationsmittel inerten Lösungsmittel, beispielsweise Dimethylformamid, durchgeführt.
Die Oxidation mit Collins-Reagenz oder Pyridiniumchlorochromat wird bei Temperaturen v on -20°C bis 50°C, vorzugsweise 0°C bis 25°C in Methylenchlorid als Lösungsmittel durchgeführt.
Die anschließende Reduktion wird nach den für den Fachmann bekannten Methoden durchgeführt, in dem das zuvor erhaltene Keton vorzugsweise mit Natriumborhydrid bei Temperaturen zwischen -40°C und +40°C, vorzugsweise -30°C bis 0°C in einem Gemisch aus Methanol und Tetrahydrofuran umgesetzt wird.

Nach chromatographischer Trennung der epimeren Alkohole erfolgt die Freisetzung der funktionell abgewandelten Hydroxygruppe nach bekannten Methoden.
Beispielsweise wird die Abspalktung der Silylschutzgruppe mittels einer organischen Säure, wie beispielsweise Oxalsäure, Essigsäure in einem mit Wasser mischbaren organischen Lösungsmittel wie z.B. Tetrahydrofuran oder mittels einer Fluorid-Verbindung, wie beispielsweise Tetrabutylöammoniumfluorid, Cäsiumfluorid, Pyridin-HF-Komplex in einem organischen Lösungsmittel, wie z.B. Tetrahydrofuran, Dioxan durchgeführt. Wenn Z in der Bedeutung 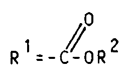
mit R²=H sein soll, so erfolgt eine Verseifung nach den dem Fachmann bekannten Methoden durch Umsatz mit einem Alkalioder Erdalkalihydroxid in einer wäßrigen Lösung eines Alkohols. Als Alkohole kommen aliphatische Alkohole in Betracht, wie z.B. Methanol, Ethanol, Butanol usw., vorzugsweise Methanol. Als Alkalihydroxyde seien Kalium-, Natrium- und Lithiumsalze genannt. Bevorzugt sind Kalium- und Lithiumsalze. Als Erdalkalihydroxyde ist beispielsweise Calsiumhydroxis geeignet. Die Umsetzung erfolft bei -10°C bis +70°C, vorzugsweise bei +25°C.

Die Reduktion zu den Verbindungen der Formel I mit R₁ in der Bedeutung einer -CH₂OH-Gruppe wird mit einem für die Reduktion von Estern oder Carbonsäuren geeigneten Reduktionsmittel wie beispielsweise Lithiumaluminiumhydrid, Diisobutylaluminiumhydrid usw. durchgeführt. Als Lösungsmittel kommen Diethylether, Tetrahydrofuran, Dimethoxyethan, Toluol usw. in Frage. Die Reduktion wird bei Temperaturen von -30 °C bis zur Siedetemperatur des verwendeten Lösungsmittels, vorzugsweise 0 °C bis 30°C vorgenommen.

Die Prostaglandinderivate der Formel I mit R₂ in der Bedeutung eines Wasserstoffatoms können mit geeigneten Mengen der entsprechenden anorganischen Base unter Neutralisierung in ein Salz überführt werden. Beispielsweise erhält man beim Lösen der entsprechenden PG-Säuren in Wasser, das die stöchiometrische Menge der Base enthält, nach Abdampfen des Wassers oder nach Zugabe eines mit Wasser mischbaren Lösungsmittels, z.B. Alkohol oder Aceton, das feste anorganische Salz.

Zur Herstellung eines Aminsalzes, die in üblicher Weise erfolgt, wird die PG-Säure z.B. in einem geeigneten Lösungsmittel, beispielsweise Ethanol, Aceton, Diethylether, Acetonitril oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die als Ausgangsmaterial dienenden Verbindungen der Formel II mit einer 15 silylgeschützten α-Hydroxygruppe können beispielsweise hergestellt werden, in dem man in an sich bekannter Weise ein nach EP 30377, EP 69696 und DE 3724190 bekanntes oder in Analogie zu synthetisierendes Diol der Formel III 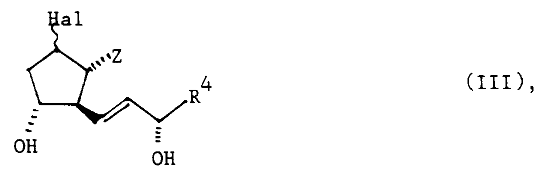
worin Z (wenn 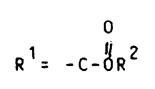
mit R² = H), Hal und R⁴ die bereits oben angegebenen Bedeutungen haben, durch Umsetzung mit einer Silylverbindung der Formel IV,

R⁵Cl (IV),

worin R⁵ die bereits oben angegebene Bedeutung hat zu den Verbindungen der Formel V überführt. 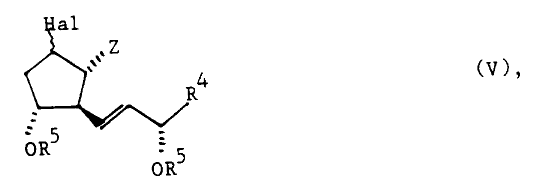
Durch eine anschließende selektive Silyletherspaltung in der 11-Position erhält man die als Ausgangsmaterial dienenden Verbindungen der Formel II mit einer 15silylgeschützten-α-Hydroxygruppe.

Die neuen Prostaglandinanaloga der Formel I sind wertvolle Pharmaka, da die bei ähnlichem Wirkungsspektrum eine wesentlich verbesserte (höhere Spezifität) und vor allem wesentlich längere Wirkung aufweisen als die entsprechenden natürlichen Prostaglandine. Zudem wird das natürliche Prostaglandin-D₂ zu dem gefäßaktiven 9α,11β-PGF₂ umgesetzt.

Die erfindungsgemäßen 9-Halogen-11β-hydroxyprostaglandinderivate der Formel I zeichnen sich durch PGD₂-typische Eigenschaften aus, d.h. sie binden gut am PGD₂-Rezeptor.

Die erfindungsgemäßen Wirkstoffe zeigen eine zytoprotektiven und ulkusheilenden Effekt, hemmen die Magensäuresekretion und wirken damit den unerwünschten Folgen nichtsteroidaler Entzündungshemmstoffe entgegen. Sie wirken außerdem an der Leber, Nieren und auch an der Bauchspeicheldrüse zytoprotektiv.

Einige der Verbindungen wirken blutdrucksendend und hemmen die Plättchenaggregation, außerdem haben sie regulierende Einflüsse auf den Herzrhythmus. Daraus ergeben sich Einsatzmöglichkeiten bei Durchblutungsstörungen zerebraler, koronarer und peripherer Art, bei Herzinfarkt bzw. zerebralem Insult und zerebralen Oedemen. Auch die Migräne ist in ihrer Symptomatik Plättchen-verursacht und damit eine wichtige Indikation.
Außerdem sind die Verbindungen zur Glaukom-Behandlung geeignet.
Aufgrund der hemmenden Wirkung auf Leukozyten, die einige der Verbindungen zeigen, und ihrer blockierenden Wirkung auf die Sauerstoffradikal-Freisetzung sind die Substanzen geeignet überschießende entzündliche Reaktionen des zellulären Immunantwort etwa bei Reperfusion oder bei Erkrankungen des rheumatischen Formenkreises zu dämpfen.

Die neuen Prostaglandine können auch in Kombination, z.B. mit β-Blockern, Diuretika, Phosphodiesterasehemmern, Calciumantagonisten, Thromboxanantagonisten, Thromboxansynthetase- und Cyclooxygenasehemmern, gerinnungshemmenden Substanzen, wie auch Fibrinolytika, Leukotrienantagonisten, Leukotriensynthetasehemmern und Antigestagenen, verwendet werden.

Diejenigen Prostaglandinanaloga, die eine hohe Affinität für Rezeptoren in Membranpräparationen aus Gehirn besitzen, können in Folge ihrer Eigenschaften zur Beeinflussung psychischer Prozesse, wie z.B. Schlaf dienen.

Die Dosis der Verbindungen ist 1-1500 »g/kg/Tag, wenn sie am menschlichen Patienten verabreicht werden.

Für die medizinische Anwendung können die Wirkstoffe in eine für die Inhalation, für orale, parenterale oder lokale (z.B. vaginale) Applikation geeignete Form überführt werden. Zur Inhalation werden zweckmäßigerweise Ärosollösungen hergestellt.

Für die orale Applikation sind beispielsweise Tabletten, Dragees oder Kapseln geeignet.

Für die parenterale Verabreichung werden sterile, injizierbare, wäßrige oder ölige Lösungen benutzt.

Fur die vaginale Applikation sind z.B. Zäpfchen geeignet und üblich.

Die Erfindung betrifft damit auch Arzneimittel auf Basis der Verbindungen der Formel I und üblichen Hilfs- und Trägerstoffe, einschließlich Cyclodextrinclathraten.

Die erfindungsgemäßen Wirkstoffe sollen in Verbindung mit den in der Galenik bekannten und üblichen Hilfsstoffen, z.B. zur Herstellung von Präparaten zur Behandlung der Hypertonie oder zur Behandlung von gastrointestinalen Störungen, wie z.B. zur Abheilung von Magen- und Zwölffingerdarmgeschwüren, dienen. Fur diesen Zweck aber auch für die übrigen Anwendungen können die Präparate 0,01 - 100 mg der aktiven Verbindung enthalten.

Die folgenden Beispiele sollen die Erfindung näher erläutern, ohne daß damit eine Begrenzung vorgenommen wird.

### Beispiel 1

### (5Z,13E)-(9R,11S,15S)-9-Chlor-15-cyclohexyl-11,15-dihydroxy-16,17,18,19,20pentanor-5,13-prostadiensäuremethylester

Zu einer Lösung von 360 mg (5Z,13E)-(9R,11R,15S)-9-Chlor-15-cyclohexyl-11hydroxy-15-tert.-butyldimethylsilyloxy-16,17,18,19,20-pentanor-5,13-prostadiensäureethylester in 150 ml Methylenchlorid gibt man bei 0°C unter Argon 4,0g Pyridiniumchlorochromat und läßt anschließend 3 Stunden bei 25°C rühren. Dann versetzt man mit Celite, filtriert, wäscht gut mit Methylenchlorid nach und engt im Vakuum ein. Man erhält 355 mg (5Z,13E)-(9R,11S,15S)-9-Chlor-15-cyclohexyl-11-oxo-15-tert.-butyldimethylsilyloxy-16,17,18,19,20-pentanor-5,13-prostadiensäuremethylester als Öl. IR (CHCl₃): 3000, 2955, 2930, 2858, 1747, 1735, 1250, 970, 838 cm⁻¹.

Das vorstehend hergestellt Keton löst man unter Argon in 15 ml Methanol/Tetrahydrofuran (2+1) und gibt bei -30°C 200 mg Natriumborhydrid hinzu. Nach 30 Minuten Rühren bei -30°C versetzt man mit wenig Eisessig und engt im Vakuum ein. Den Rückstand nimmt man in Wasser auf, extrahiert dreimal mit Methylenchlorid, wäscht die vereinigten organischen Phasen mit Sole, trocknet über Natriumsulfat ujnd engt nach der Filtration im Vakuum ein. Das so erhaltene Rohprodukt wird an Kieselgel chromatographiert. Mit Hexan 10-20% Essigester erhält man neben dem polareren 11-α-Epimeren Alkohol 94 mg des unpolareren (5Z,13E)-(9R,11S,15S)-9-Chlor-15-cyclohexyl-11-hydroxy-15-tert.-butyldimethylsilyloxy-16,17,18,18,19-pentanor-5,13-prostadiensäuremethylester als farbloses Öl. IR (CHCl₃): 3610, 3500, 3000, 2955, 2930, 2855, 1733, 1250, 975, 848 cm⁻¹.

Der vorstehend hergestellte 11β-Alkohol wird in einem Gemisch aus Essigsäure/Wasser/Tetrahydrofuran (65+35+10) für 6 Stunden auf 40°C erwärmt und anschliessend für 14 Stunden bei 24°C weiter gerührt. Unter Toluolzusatz wird im Vakuum eingeengt und der so erhaltene Rückstand an Kieselgel chromatographiert. Mit Hexan /0-80% Essigester erhält man 55 mg der Titelverbindung als farbloses Öl.
IR (CHCl₃): 3605, 3430, 3000, 2950, 2928, 2858, 1730, 978 cm⁻¹.
Der als Ausgangsmaterial verwendete 15-Homosilylether wird wie folgt erhalten:
1a) (5Z,13E)-(9R,11R,15S)-9-Chlor-15-cyclohexyl-11,15-bis-tert.-butyldimethylsilyloxy-16,17,18,19,20-pentanor-5,13-prostadiensäuremethylester
   Zu einer Lösung von 600 mg (5Z,13E)-(9R,11R,15S)-9-Chlor-15-cyclohexyl11,15-dihydroxy-16,17,18,19,20-pentanor-5,13-prostadiensäuremethylester in 10 ml Dimethylformamid gibt man bei 0°C unter Argon 1g Imidazol und 1,1g tert.-Butyldimethylsilylchlorld und rührt 24 Stunden bei 25°C. Anschließend gibt man die Reaktionslösung auf Wasser und extrahiert mehrmals mit Hexan/Ether (1+1). Die vereinigten organischen Phasen wäscht man einmal mit Wasser, trocknet über Magnesiumsulfat, filtriert und engt im Vakuum ein. Das so erhaltene Rohprodukt reinigt man durch Chromatographie an Kieselgel. Mit Hexan/0-10% Essigester erhält man 584 mg der Titelverbindung als farbloses Öl.
   IR (CHCl₃): 3000, 2952, 2930, 2858, 1730, 1250, 975, 848 cm⁻¹.
1b) (5Z,13E)-(9R,11R,15S)-9-Chlor-15-cyclohexyl-11-hydroxy-15-tert.-but yl-dimethylsilyloxy-16,17,18,19,20-pentanor-5,13-prostadiensäuremethylester
   Zu einer Lösung von 580 mg des vorstehend hergestellten Bis-silylethers in 5 ml Tetrahydrofuran gibt man bei 25°C unter Argon 20 ml einer 1molaren Lösung von Tetrabutylammoniumfluorid in Tetrahydrofuran und rührt 30 Minuten bei 25°C. Anschließend verdünnt man mit 100 ml Ether, wäscht dreimal mit Wasser, trocknet über Natriumsulfat und engt nach Filtration im Vakuum ein. Den so erhaltenen Rückstand chromatographiert man an Kieselgel. Mit Hexan/0-10% Essigester erhält man 382 mg der Titelverbindung als faroloses Öl. IR (CHCl₃): 3605, 3520, 3000, 2955, 2930, 2858, 1732, 1252, 975, 848 cm⁻¹

### Beispiel 2

### (5Z,13E)-(9R,11S,15S)-9-Chlor-15-cyclohexyl-11,15-hydroxy-3-oxa-16,17,18,19,20-pentanor-5,13-porostadiensäure-tert.-butylester

In Analogie zu Beispiel 1 erhält man aus 360 mg (5Z,13E)-(9R,11R,15S)-9-Chlor15-cyclohexyl-11-hydroxy-3-oxa-15-bis-tert.-butyldimethylsilyoxy-16,17,18,19,20-pentanor5,13-prostadiensäure-tert.-butylester 98 mg der Titelverbindung als farbloses Öl.
IR (CHCl₃): 3600, 3415, 3200, 2988, 2930, 2858, 1742, 1238, 982 cm⁻¹

Das Ausgangsmaterial für die Herstellung der Titelverbindung erhält man gemäß Beispiel 1a,b) aus 500 mg (5Z,13E)-(9R,11R,15S)-9-Chlor-15-cyclohexyl-11,15dihydroxy-3-oxa-16,17,18,19,20-pentanor-5,13prostadiensäure-tert.-butylester.

### Beispiel 3

### (5Z,13E)-(9R,11S,15S)-9-Fluor-15-cyclohexyl-11,15-dihydroxy-16,17,18,19,20-pentanor-5,13-porostadiensäuremethylester

In Analogie zu Beispiel 1 erhält man aus 1,03 g (5Z,13E)-(9R,11R,15S)-9-Fluor15-cyclohexyl-11-hydroxy-15-tert.-butyldimethylsilyloxy-16,17,18,19,20-pentanor5,13-prostadiensäuremethylester 210 mg der Titelverbindung als farbloses Öl.
IR (CHCl₃): 3645, 3605, 3420, 3000, 2950, 2925, 2855, 1730, 1245, 975 cm⁻¹

Das Ausgangsmaterial für die Herstellung der Titelverbindung erhält man gemäß Beispiel 1a,b) aus 1,48 g (5Z,13E)-(9R,11R,15S)-9-Fluor-15-cyclohexyl-11,15dihydroxy-16,17,18,19,20-pentanor-5,13-prostadiensäuremethylester.

### Beispiel 4

### (5Z,13E)-(9R,11S,15S)-9-Chlor-15-cyclohexyl-11,15-dihydroxy-16,17,18,19,20-pentanor-5,13-prostadiensäure

52 mg des nach Beispiel 1 hergestellten Esters rührt man in 4ml eines Gemisches aus Kaliumhydroxid in Ethanol/Wasser (2g KOH in 100 ml EtoH/H₂O 3+1) für 4 Stunden bei 25°C. Dann säuert man mit verdünnter Salzsäure bis pH=5 an, extrahiert mehrmals mit Essigester, wäscht die vereinigten organischen Phasen mit Sole, trocknet über Nariumsulfat und engt nach Filtration im Vakuum ein. Den so erhaltenen Rückstand chromatographiert man an Kieselgel. Mit Methylenchlorid/ 0-70% Aceton erhält man 33 mg der Titelverbindung als farbloses Öl.
IR (CHCl₃): 3600, 3400, 3000, 2950, 2928, 2855, 1712, 978 cm⁻¹

### Beispiel 5

### (5Z,13E)-(9R,11S,15S)-9-Chlor-15-cyclohexyl-11,15-dihydroxy-3-oxa-16,17,18,19,20-pentanor-5,13-prostadiensäure

95 mg des nach Beispiel 2 hergestellten Esters gelöst in 0,85 ml Methanol versetzt man mit 10,3 mg Lithiumhydroxid gelöst in 0,85 ml Wasser und rührt 18 Stunden bei 25°C. Dann säuert man mit verdünnter Salzsäure bis pH=5 an, extrahiert mehrmals mit Essigester, wäscht die vereinigten organischen Basen mit Sole, trocknet über Nariumsulfat und engt nach Filtration im Vakuum ein. Den so erhaltenen Rückstand chromatographiert man an Kieselgel. Mit Hexan/70% Essig-ester/ 0-5% 2-Propanol erhält man 81 mg der Titelverbindung als farbloses Öl.
IR (Film): 3420, 3020, 2970, 2925, 2850, 1732, 978 cm⁻¹

### Beispiel 6

### (5Z,13E)-(9R,11S,15S)-9-Fluor-15-cyclohexyl-11,15-dihydroxy-16,17,18,19,20-pentanor-5,13-prostadiensäure

In Analogie zu Beispiel 4 erhält man aus 204 mg des nach Beispiel 3 hergestellten Esters 91 mg der Titelverbindung als farbloses Öl.
IR (CHCl₃): 3680, 3600, 3420, 3030, 3000, 2960, 2925, 2855, 1710, 976 cm⁻¹

## Patentansprüche

1. 9-Halogen-11β-hydroxy-prostanderivate der Formel I 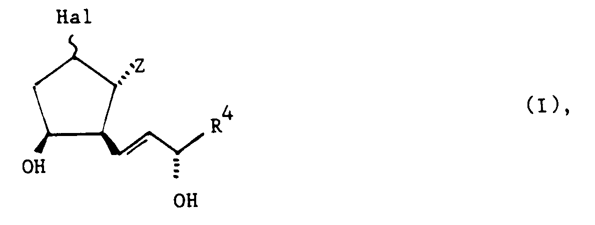 worin Z die Reste 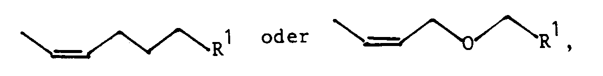 Hal ein α- oder β-ständiges Chlor- oder Fluoratom,
R¹ den Rest CH₂OH oder 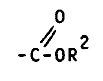 mit R² in der Bedeutung eines Wasserstoffatoms, eines Alkyl-, Cycloalkyl-, Aryl- oder heterocyclischen Restes oder R¹ den Rest 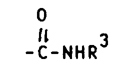 mit R³ in der Bedeutung eines Säurerestes oder des Restes R² und R⁴ eine Cycloalkylgruppe, und falls R² die Bedeutung eines Wasserstoffatoms hat, deren Salze mit physiologisch verträglichen Basen bedeuten und deren Cyclodextrinclathrate.

2. Verfahren zur Herstellung von erfindungsgemäßen 9-Halogen-11β-hydroxyprostanderivate der Formel I, dadurch gekennzeichnet, daß man in an sich bekannter Weise eine Verbindung der Formel II 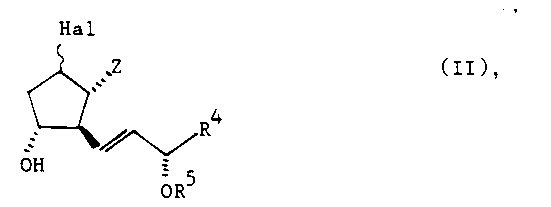 worin Z, Hal und R⁴ die bereits oben angegebenen Bedeutungen haben und R⁵ ein Silylrest sein kann durch Oxidation mit Chromsäure oder einem Chromsäurederivat intermediar in ein Keton überführt, dieses anschließend durch Reduktion in ein Gemisch der epimeren Alkohole umsetzt und gegebenenfalls in beliebiger Reihenfolge vorhandene Schutzgruppen abspaltet und/oder eine veresterte Carboxylgruppe 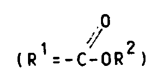 verseift und/oder reduziert wird.

3. Arzneimittel, enthaltend eine oder mehrere Verbindungen der Formel I und übliche Hilfs- und Trägerstoffe.

## Claims

1. 9-Halo-11β-hydroxy-prostane derivatives of the formula I 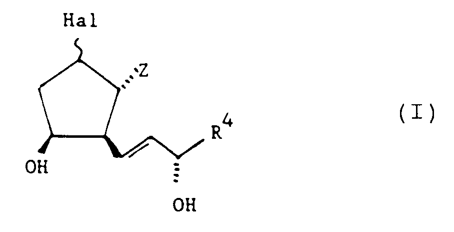 wherein
Z is a radical 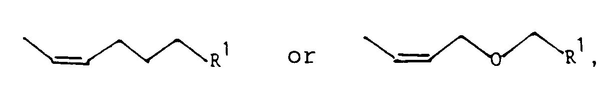
Hal is a chlorine or fluorine atom in the α- or β-configuration,
R¹ is the radical CH₂OH or 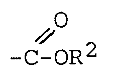 with R² being a hydrogen atom or an alkyl, cycloalkyl, aryl or heterocyclic radical, or 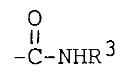
R¹ is the radical with R³ being an acid radical or having the same meaning as given for the radical R², and
R⁴ is a cycloalkyl group,
and, if R² is a hydrogen atom, their salts with physiologically acceptable bases and their cyclodextrin clathrates.

2. A process for the preparation of 9-halo-11β-hydroxyprostane derivatives of the formula I according to the invention, characterised in that in a manner known per se a compound of the formula II 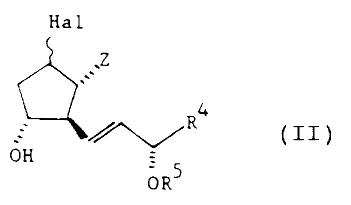 wherein Z, Hal and R⁴ have the meanings already given above and R⁵ may be a silyl radical, is converted by oxidation with chromic acid or with a chromic acid derivative intermediately into a ketone, which is then converted by reduction into a mixture of the epimeric alcohols and optionally, in any desired order, any protecting groups present are removed and/or an esterified carboxy group 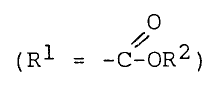 is hydrolysed and/or reduced.

3. Medicaments comprising one or more compounds of the formula I and customary excipients and carriers.

## Revendications

1. Dérivés de 9-halogéno-11β-hydroxy-prostane de formule I 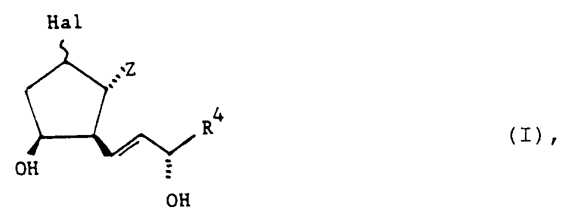 dans laquelle Z signifie les restes 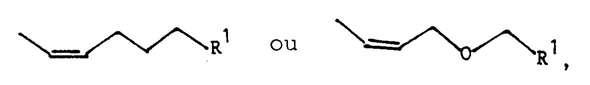 Hal signifie un atome de chlore ou de fluor en position α ou β,
R¹ signifie les restes CH₂OH ou 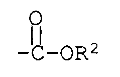 avec R² ayant la signification d'un atome d'hydrogène, d'un reste alkyle, cycloalkyle, aryle ou d'un reste hétérocyclique ou R¹ signifie le reste 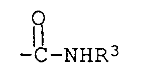 avec R³ ayant la signification d'un reste d'acide ou du reste R² et R⁴ signifie un groupe cycloalkyle, leurs sels de base compatibles du point de vue physiologique dans le cas où R² signifie un atome d'hydrogène, ainsi que leurs clathrates à base de cyclodextrine.

2. Procédé de préparation des dérivés 9-halogéno-11β-hydroxy-prostane de formule I conformes à l'invention, caractérisé en ce qu'on transforme, d'une manière connue en soi, un composé de formule II 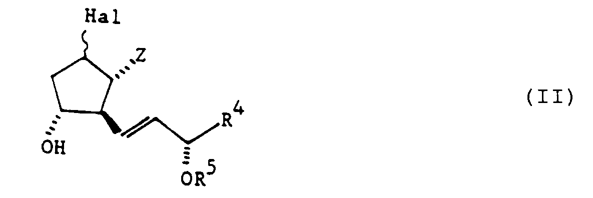 dans laquelle Z, Hal et R⁴ ont la signification déjà donnée ci-dessus et R⁵ peut être un reste silyle, intermédiairement en une cétone par une oxydation avec l'acide chromique ou avec un dérivé de l'acide chromique, cette cétone est ensuite transformée en un mélange d'alcools épimères par une réduction et, les groupements protecteurs sont éliminés éventuellement selon un ordre quelconque et/ou un groupe carboxyle 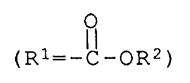 estérifié et saponifié et/ou réduit.

3. Médicament, contenant un ou plusieurs composés de formule I et des substances supports et auxiliaires classiques.
